# EUROPEAN PATENT SPECIFICATION

(11) **EP 2 370 067 B1**
(45) Date of publication and mention of the grant of the patent: **06.11.2013**
(21) Application number: 09798876.0
(22) Date of filing: 10.12.2009
(51) Int. Cl.: A61K 9/70, A61K 31/52, A61K 31/522, A61K 38/00, A61K 38/21

(54) **ANTIVIRAL PATCH**
ANTIVIRAL WIRKENDES PFLASTER
PATCH ANTIVIRAL

(30) Priority: 15.12.2008 EP 08171696
(43) Date of publication of application: 05.10.2011
(73) Proprietor: Bouty S.p.A., 20129 Milano (IT)
(72) Inventor: MARRA, Fabio, 20129 Milano (IT); Dl GRIGOLI, Maurizio, 20129 Milano (IT); COMUZIO, Sergio, 20129 Milano (IT)
(74) Representative: Minoja, Fabrizio
(86) International application number: PCT/EP2009/008843
(87) International publication number: WO 2010/078911

(56) References cited:
- DE-A1-102005 003 387
- DE-A1-102005 050 654

## Description

The invention refers to a patch as defined by the claims comprising a skin adhesive layer, a backing layer and a release liner, the adhesive layer comprising an antiviral agent dissolved in a low volatile solvent and a polymeric adhesive soluble in highly volatile solvents.

### Background of the invention

Herpes simplex labialis (HSL) is characterized by recurrent cold sores resulting primarily from herpes simplex virus type 1 infection of the lips and perioral skin. HSL is prevalent, occurring in approximately 15% to 40% of adults worldwide (1-4).

Topical antiviral therapies, such as acyclovir and penciclovir cream or ointment, are commonly used for treating subjects with HSL (5,6), but overall benefits of these therapies may be limited by several factors, including the need for frequent application, lack of protection of the lesion, formation of a scar and aesthetic factors relating to the visibility of the drug product on the site of application or appearance of the lesion during the healing process.

Many of the limitations of standard topical antiviral therapy for the treatment of HSL lesions may be addressed by appropriate wound management. Recent clinical evidence has suggested that HSL lesions are deeper than epidermal wounds and may be classified as partial-thickness wounds that extend through the basal membrane into the dermis (7).

Protection of HSL lesions from foods or fluids, general mouth activity and external environmental factors may be critical in reducing pain and promoting wound re-epithelization and proper healing.

According to a recent study, 61 % of 998 health care professionals, who treat recurrent HSL, reported that the social embarrassment of having a cold sore was a major concern (8).

Likewise, a recent comprehensive review of psycho-dermatologic disorders listed herpes simplex virus infection as a disorder associated with a variety of psychopathologic problems that may be exacerbated by emotional stress (9).

One or more drug treatments for skin administration, including acyclovir cream and ointment, penciclovir cream and idoxuridine in dimethyl sulfoxide (DMSO) (10), are commercially available, but their administration is inconvenient, needing frequent applications for several days, especially in the early stage of the development of the pathology. Moreover the visibility of the product can reduce the patients compliance.

On the other side, a number of treatments are available over the counter, comprising either active ingredients reducing lesion pain or promoting wound healing through occlusion, without any anti-viral agent, such as for instance the product Compeed^{®} marketed by Johnson & Johnson (11).

A mucoadhesive patch for the treatment of diseases of any body cavity, such as mouth, was already described in US 2008/0274164. Atrium patch, according to the definition given in said US patent application, is a mucoadhesive patch, comprising organic polymers and at a least a drug, that can be included in one of the two layers composing it. The patch is realized by casting the first adhesive layer onto any adequate smooth surface and dried. The second layer is not adhesive and is layered on the first by a further casting procedure. According to this procedure, two consequent casting steps are necessary. The essential feature of this product is that moisture is necessary to exert adhesion on the site of application, because of the very peculiar blend of polymers in the adhesive matrix. As described in one of the embodiments, Atrium patch was also intended for anti-herpetic activity. The patients were invited to wet the lips with saliva by the tongue before Atrium patch application. This not desirable procedure, that can increase the risk of the diffusion of the viral lesions, was necessary because Atrium patch is not self adhesive: the adhesion is obtained only in certain moisture condition of the site of application.

### Description of the invention

Each of the treatments and the previous patents, representative of the prior art discussed briefly above, has certain disadvantages as compared to the present invention.

The present invention refers to a patch to treat and simultaneously to cover and protect the herpes viral lesions.

More particularly, the present invention provides a self-adhesive patch useful to deliver continuously anti-viral drugs to the sites of the body that could be normally infected by Herpes Simplex or by Herpes Zoster Viruses.

The use of the patch of the invention is very practical for the patient. The patch is effective in delivering drugs to the skin and compared to the conventional creams or ointments, it provides a continuous drug delivery without repeated daily applications.

Moreover, actually marketed formulations consist in drug suspensions, in which solid particles of drug are dispersed into the drug product.

Now, we found surprisingly that any antiviral agent can be dissolved in a patch, under peculiar conditions. The bioavailability of a drug is improved when it is dissolved in a suitable vehicle, granting a higher diffusivity across the skin and increasing the amount that can reach the site to treat.

The application of creams and ointment implies the contact with the infected area. Moreover, they are not cosmetically accepted by the patients. The present invention is safer in the application and plays a cosmetic role by masking cold sores, which helps the patient also from the psychological point of view.

The present invention overcomes the disadvantages of a mucoadhesive patch, providing a self-adhesive composition, easily manufactured by just a single casting procedure, and able to stick on the site affected by the viral infection in any moisture conditions. The moistening of the infected area will no longer be necessary before applying the patch of the invention, thus reducing the risks of contamination and of further infections.

More particularly, the patch of the invention comprises a skin adhesive layer, a backing layer and a release liner, the adhesive layer comprising an antiviral agent dissolved in a low volatile solvent and a polymeric adhesive soluble in highly volatile solvents as defined by the claims.

The antiviral agent is selected from acyclovir, penciclovir, famciclovir, idoxuridine, edoxudine; more preferably acyclovir, pencivlovir or famciclovir.

Said antiviral agent may be incorporated in the adhesive layer in an amount from 0.1 to 10% by weight of the dried adhesive layer, dissolved in a low volatile solvent.

According to different authors, as stated in different books and papers (12-15), the solvents can be classified in function of their physical-chemical properties. Key properties, among the others, include density, viscosity, dielectric constant, dipole moment, melting and boiling point.

Solvents can be broadly classified as low, middle or highly boiling according to boiling temperature at 1 bar:
■ Low Boiling: Boiling ranges below 100°C
■ Medium Boiling: Boiling ranges between 100°C and 150°C
■ High Boiling: Boiling ranges above 150°C

For comparison, the boiling point of water is 100°C at 1 bar.

Small amounts of low-boiling solvents, like diethyl ether, dichloromethane, or acetone, will evaporate in few seconds at room temperature, while high-boiling solvents like xylene or dimethyl sulfoxide need higher temperatures, an air flow, or the application of vacuum for fast evaporation.

In this meaning, a low boiling solvent is a highly volatile solvent whereas a high boiling solvent is a solvent with a poor inclination to evaporate so that it can be defined as a low volatile solvent.

The low volatile solvent according to the invention is dimethylsulfoxide,

The low volatile solvent is preferably present in an amount from 10 to 50% by weight of the dried adhesive layer.

The adhesive polymer is selected from pectin, agar gum, acacia gum, xanthan gum, polyvinyl alcohol, polymethacrylic acid, polymethacrylate, acrylates/alkylmethacrylates copolymers, any acrylic ester copolymer, aminoalkyl methacrylate copolymer, polyvinyl pyrrolidone, cellulose or cellulose derivatives, such as hydroxypropylcellulose, hydroxyethylcellulose, or blends thereof.

The adhesive layer is formed from a solution of the adhesive polymer in a highly volatile solvent, i.e having a low boiling point (in the range of 40°C-100°C) and high vapor pressure. Said solvent is then usually evaporated during the manufacturing process even though a certain amount, up to 15% by weight, may be left in the adhesive layer after drying.

The adhesive polymer or the adhesive polymer blend is present in an amount from 20 to 50% by weight of the dried adhesive layer.

The patch may further contain citric acid, succinic acid, lactic acid and esters thereof as a non polymeric crystallization inhibitors, in an amount from 0.5 to 15% by the weight of the dried adhesive layer.

The patch may also contain other excipients such as cross-linkers, penetration enhancers, plasticizers, preservatives, antioxidants, fragrances, emollients.

The backing layer is transparent, semi-occlusive or occlusive, oxygen permeable, preferably consisting of polyurethane ether or ester film, polyethylene, ethylene vinyl acetate or polyolephine film with a MVTR (moisture vapor transmission rate) from 50 to 3500 g/m²/day and a thickness from 20 to 150 µm.

The backing layer is very flexible and soft, transparent or colored and can be occlusive or perspirating, providing a masking effect of the cold sore. Moreover, it protects the damaged skin and the viral lesions from the external contact, thus reducing the patient's pain and the possibility of further contaminations or infections, and improving the re-epithelization process.

The adhesive layer is protected from the external environment through a release liner, that has to be removed before applying the patch to the site of the body interested by the viral lesions. Once the patch is applied, through the self-adhesive layer, it can be kept on-site up to 12 hours, delivering the active ingredient into and across the skin.

The patches of the invention are prepared by a process comprising the steps of blending the solution of adhesive polymers in highly volatile solvents together with the other components and then casting the mixture on a silicone coated liner film, before drying and the final lamination.

The highly volatile solvents evaporate, leaving the adhesive film on the release liner whereas the low volatile solvent remains in the adhesive layer preventing the drug crystallization.

The polymers used according to this invention are those normally used to produce pressure sensitive adhesives (PSAs) or bio-adhesive film in an organic or aqueous solution, in a concentration ranging from 20% to 80%, preferably from 20 to 50% of the composition of the adhesive mixture, while the concentrations of the highly volatile solvent are from 10% to 50%. Other components of the adhesive layer or of the reservoir layer include thickening agents, chemical permeation enhancers, non-polymeric crystallization inhibitors, flavours, surfactants, cross-linkers, buffering agents, plasticizers, preservatives, anti-oxidants, pigments.

The selected solvents and polymers must of course be compatible and form an homogeneous solution which may be uniformly casted.

Low boiling point solvents, i.e. high volatile solvents with boiling point not higher than 100°C, are preferably water, ethanol, methanol, isopropyl alcohol, ethyl acetate, more preferably water.

This invention actually allows to produce an anti-herpes patch, having an effective amount of anti-viral drug that can be continuously delivered to the site of application.

The low volatile solvents helps to avoid crystallization by keeping dissolved the active substances in the matrix and affects the diffusivity of the drug through the matrix, to reach the skin and the site of action. The matrix must be chosen according to the physical-chemical properties of the low volatile solvents or the solvents blend. The polymer must provide a good cohesiveness to the final product. The quantitative composition of the adhesive blend is chosen in order to have an acceptable film in terms of thickness, cohesion properties, mechanical resistance, skin adhesion, peel properties and handling.

The polymer blend range in the dry matrix is 5%-50%, most preferably 20%-35%, solubilized in a low boiling point solvent or solvent mixture. The solvent percentage ranges from 20% to 70%, preferably from 35% to 55%, in the mixture that has to be casted to produce the adhesive layer or the reservoir layer. In the dry matrix the amount of low boiling point solvent must not exceed 15% by weight.

The low volatile solvents, instead, are included in the dry matrix, entangled in the polymer and dissolving the active ingredients. The amount of these solvents in the dry state is in the range 10%-50%, but preferably in the range 30%-55%.

The invention is described in more detail in the following examples.

### Example 1 - ACV019FM composition

A patch formulation comprising 3.7% of acyclovir in the dry matrix and employs as adhesive a blend of PVP (Kollidon^{®} K 90 P) and a water solution of acrylates/dimethylamino methacrylates copolymer, known as Plastoid^{®}. Table 1 reports the composition of the formulation. Acyclovir is solubilized in dimethyl sulfoxide, known also to improve permeation of active substances through the skin.

The transparent solution is mixed to the adhesive blend, and, once homogeneity is reached, the mass is film-casted on the release liner and dried in oven.

The patch is completed by laminating the backing layer on the adhesive layer and cutting in the opportune dimension and shape.

**Table 1: ACV019FM composition**

| **Ingredients** | **% wet matrix** | **% dry matrix** |
|---|---|---|
| Water (volatile solvent) | **40.5** | - |
| Water from Plastoid^{®} (volatile solvent) | **12.0** | - |
| PEG 400 (plasticizer) | **7.4** | **14.1** |
| DMSO (low volatile solvent) | **22.7** | **43.5** |
| PVP K 90 P (adhesive polymer component) | **13.1** | **25.0** |
| Citric acid (crystallization inhibitor) | **2.5** | **4.8** |
| Acyclovir | **1.9** | **3.7** |
| Plastoid solid (adhesive polymer blend) | **4.6** | **8.9** |
| TOT | 100.00 | 100.0 |

### Example 2 - PEN001FM

A patch containing penciclovir is described. The preparation steps are similar to the previous. Table 2 reports the composition of the formulation.

**Table 2: PEN001FM composition**

| **Ingredients** | **% wet matrix** | **% dry matrix** |
|---|---|---|
| Water (volatile solvent) | **45.4** | - |
| PEG 400 (plasticizer) | **6.8** | **11.9** |
| DMSO (low volatile solvent) | **26.6** | **46.6** |
| PVP K 90 P (adhesive polymer) | **14.2** | **25.0** |
| Diethyl citrate (crystallization inhibitor) | **6.2** | **11.0** |
| Penciclovir | **0.3** | **1.4** |
| Polyvinyl alcohol (adhesive polymer blend) | **2.3** | **4.1** |
| TOT | 100.00 | 100.0 |

### Example 3 - Transepidermal permeation of acyclovir through separated epidermis of pig ear skin

In vitro permeation of the acyclovir formulations was performed over epidermis obtained from porcine inner ear skin, using Franz diffusion cells and a degassed phosphate-buffered saline solution (PBS) as receptor solution. A 5% acyclovir commercial cream was employed as reference (Cycloviran^{®}). Drug permeation rate through the epidermis and the amount of acyclovir accumulated at the end of the experiments were evaluated by HPLC analysis.

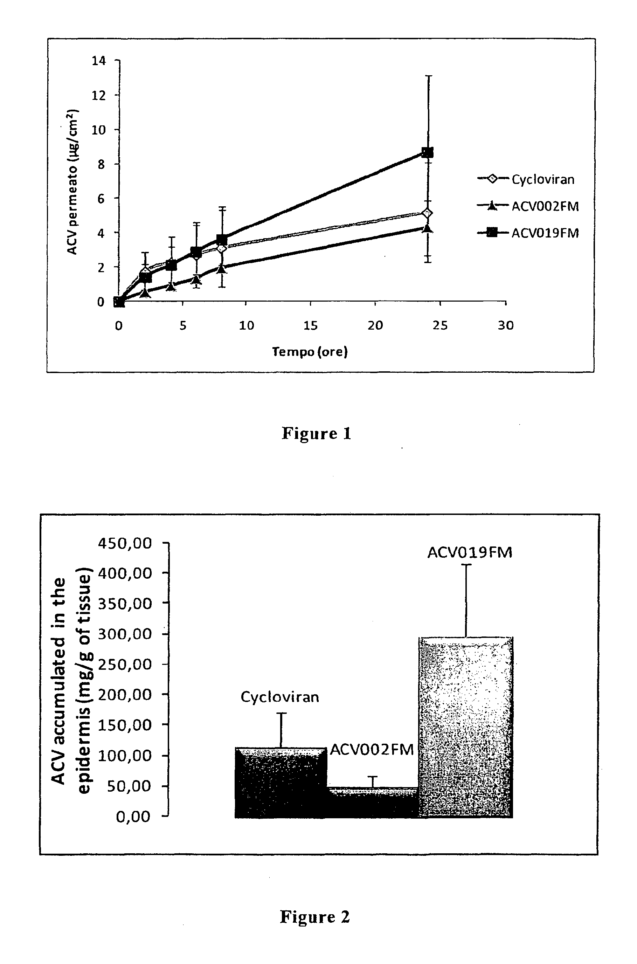

Figure 1 describes the permeation profile of acyclovir through porcine epidermis, comparing the drug permeated from two patches with the amount permeated from the commercial reference.

Acyclovir permeation rate across the epidermis from the formulation ACV002FM is not significantly different from the amount of drug permeated from the commercial reference formulation, while the permeation profile of acyclovir generated following the application of ACV019FM is significantly higher, being the patch more effective in delivering acyclovir across the skin.

In figure 2 the amount of acyclovir recovered in the skin after the permeation experiment is reported.

The data analysis also shows that the amount of drug recovered in the epidermis after 24 hours of application of ACV002FM is not significantly different from Cycloviran^{®}. On the other side, ACV019FM provided higher amount of ACV in the epidermis than the reference.

### References

1) Embil JA, Can Med Assoc J 1975; 113: 627-630.
2) Lowhagen GB, Scand J Infect Dis 2002; 34: 664-667.
3) Malvy D, et al. J Eur Acad Dermatol Venereol 2007; 21: 1398-1403.
4) Young TB, Am J Epidemiol 1988; 127: 612-625.
5) Spruance SL, Antimicrob Agents Chemother 2002; 46: 2238-2243.
6) Raborn GW, et al.. J Am Dent Assoc 2002; 133: 303-309.
7) Patel AR, Adv Skin Wound Care 2007; 20: 408-412.
8) Raborn GW, J Am Dent Assoc 2004; 135: 48-54.
9) Jafferany M. Prim Care Companion J Clin Psychiatry 2007; 9: 203-213.
10) Hamuy R, Eur J Derm, 1998; 8: 310-319.
11) Karlsmark T et al, JEADV 2008: 1-9.
12) Reichardt C, Solvents and Solvent Effects in Organic Chemistry, VCH, Weinheim, 2nd edition, 1988.
13) Wypych G, editor, Handbook of Solvents, Willian Andrew-Chemtec Publishing, 2001.
14) Adams D J et al, Chemistry in alternative reaction media, Wiley Science, 2004.
15) Gramatica P et al, TrAC 1999, 7: 461-471.

## Claims

1. A patch comprising a skin self-adhesive layer, a backing layer and a release liner, the adhesive layer comprising an antiviral agent selected from the group consisting of acyclovir penciclovir, famciclovir, idoxuridine, edoxudine dissolved in dimethylsulfoxide and a polymeric adhesive soluble in highly volatile solvents.

2. The patch of claim 1, wherein the content of an antiviral agent is from 0.1 to 10% by weight of the dried adhesive matrix.

3. The patch of claim 1 or 2, wherein the content of dimethylsulfoxide is from 10 to 50% by weight of the dried adhesive matrix.

4. The patch of any one of claims 1-3, wherein the adhesive polymer is selected from pectin, agar gum, acacia gum, xanthan gum, polyvinyl alcohol, polymethacrylic acid, polymethacrylate, acrylates/alkylmethacrylates copolymers, any acrylic ester copolymer, aminoalkyl methacrylate copolymer, polyvinyl pyrrolidone, cellulose or cellulose derivatives, such as hydroxypropylcellulose, hydroxyethylcellulose, or blends thereof.

5. The patch of any one of claims 1-4, wherein the adhesive polymer or the adhesive polymer blend is present in an amount from 20 to 80% by weight of the dried adhesive matrix.

6. The patch of any one of claims 1-5, further containing a non polymeric crystallization inhibitor selected from citric acid, succinic acid, lactic acid, and esters thereof, in an amount from 0.5 to 15% by the weight of the dried adhesive matrix.

7. The patch of any one of claims 1-6, further containing any other suitable excipients selected from cross-linkers, penetration enhancers, preservatives, plasticizers, fragrances, antioxidants and emollients.

8. The patch of any one of claims 1-7, wherein the backing layer is transparent, semiocclusive or occlusive, oxygen permeable.

9. The patch according to claim 8, wherein the backing layer is polyurethane ether or ester film, polyethylene, ethylene vinyl acetate or polyolephine film with a MVTR (moisture vapor transmission rate) from 50 to 3500 g/m²/day and a thickness from 20 to 150 µm.

10. The patch of any one of claims 1-9, wherein the highly volatile solvent with boiling point not higher than 100°C is selected from water, ethanol, acetone, isopropanol, acetic acid, isobutanol, ethyl acetate, methyl ethyl ketone.

## Patentansprüche

1. Pflaster, das eine hautselbstklebende Schicht, eine Trägerschicht und eine abziehbare Schutzfolie umfasst, wobei die Klebschicht ein antivirales Mittel, ausgewählt aus der Gruppe, bestehend aus Acyclovir, Penciclovir, Famciclovir, Idoxuridin, Edoxudin, gelöst in Dimethylsulfoxid, und ein polymerisches Klebemittel, das in leicht flüchtigen Lösungsmitteln löslich ist, umfasst.

2. Pflaster nach Anspruch 1, wobei der Gehalt an antiviralem Mittel 0,1 bis 10% nach Gewicht der getrockneten Klebmatrix beträgt.

3. Pflaster nach Anspruch 1 oder 2, wobei der Gehalt an Dimethylsulfoxid 10 bis 50% nach Gewicht der getrockneten Klebmatrix beträgt.

4. Pflaster nach einem jeglichen der Ansprüche 1-3, wobei das Klebemittelpolymer aus Pektin, Agargummi, Gummi arabicum, Xanthan, Polyvinylalkohol, Polymethacrylsäure, Polymethacrylat, Acrylaten/Alkylmethacrylaten-Copolymeren, einem jeglichen Acrylester-Copolymer, Aminoalkylmethacrylat-Copolymer, Poly-vinylpyrrolidon, Cellulose oder Cellulosederivaten, wie Hydroxypropylcellulose, Hydroxyethylcellulose, oder Gemischen davon ausgewählt ist.

5. Pflaster nach einem jeglichen der Ansprüche 1-4, wobei das Klebemittelpolymer oder das Klebemittelpolymergemisch in einer Menge von 20 bis 80% nach Gewicht der getrockneten Klebmatrix vorhanden ist.

6. Pflaster nach einem jeglichen der Ansprüche 1-5, das ferner einen nicht polymerischen Kristallisationshemmstoff, ausgewählt aus Citronensäure, Bernsteinsäure, Milchsäure und Estern davon, in einer Menge von 0,5 bis 15% nach Gewicht der getrockneten Klebmatrix enthält.

7. Pflaster nach einem jeglichen der Ansprüche 1-6, das ferner jegliche andere geeignete Exzipienzien, ausgewählt aus Vernetzern, Penetrationsverstärkern, Konservierungsmitteln, Weichmachern, Parfümen, Antioxidationsmitteln und Aufweichmitteln, enthält.

8. Pflaster nach einem jeglichen der Ansprüche 1-7, wobei die Trägerschicht transparent, semiokklusiv oder okklusiv, sauerstoffpermeabel ist.

9. Pflaster gemäß Anspruch 8, wobei die Trägerschicht ein Polyurethanether- oder -esterfilm, Polyethylen-, Ethylenvinylacetat- oder Polyolefinfilm mit einem Wert für MVTR (Wasserdampfdurchlässigkeit) von 50 bis 3500 g/m²/Tag und einer Dicke von 20 bis 150 µm ist.

10. Pflaster nach einem jeglichen der Ansprüche 1-9, wobei das leicht flüchtige Lösungsmittel mit einem Siedepunkt von nicht mehr als 100°C aus Wasser, Ethanol, Aceton, Isopropanol, Essigsäure, Isobutanol, Ethylacetat, Methylethylketon ausgewählt ist.

## Revendications

1. Patch comprenant une couche auto-adhésive sur la peau, une couche de support et une couche à détacher, la couche adhésive comprenant un agent antiviral choisi dans le groupe constitué par l'acyclovir, le penciclovir, le famciclovir, l'idoxuridine, l'édoxudine dissous dans du diméthylsulfoxyde et un adhésif polymère soluble dans les solvants très volatils.

2. Patch selon la revendication 1, dans lequel la teneur d'un agent antiviral est de 0,1 à 10 % en poids de la matrice adhésive séchée.

3. Patch selon la revendication 1 ou 2, dans lequel la teneur de diméthylsulfoxyde est de 10 à 50 % en poids de la matrice adhésive séchée.

4. Patch selon l'une quelconque des revendications 1 à 3, dans lequel le polymère adhésif est choisi parmi la pectine, la gomme d'agar, la gomme arabique, la gomme de xanthane, l'alcool polyvinylique, l'acide polyméthacrylique, le polyméthacrylate, les copolymères d'acrylates/alkylméthacrylates, n'importe quel copolymère d'ester acrylique, un copolymère de méthacrylate d'aminoalkyle, la polyvinylpyrrolidone, la cellulose ou les dérivés de cellulose, tels que l'hydroxypropylcellulose, l'hydroxyéthylcellulose, ou les mélanges de ceux-ci.

5. Patch selon l'une quelconque des revendications 1 à 4, dans lequel le polymère adhésif ou le mélange de polymère adhésif est présent en une quantité de 20 à 80 % en poids de la matrice adhésive séchée.

6. Patch selon l'une quelconque des revendications 1 à 5, contenant en outre un inhibiteur de cristallisation non polymérique choisi parmi l'acide citrique, l'acide succinique, l'acide lactique, et les esters de ceux-ci, en une quantité de 0,5 à 15 % en poids de la matrice adhésive séchée.

7. Patch selon l'une quelconque des revendications 1 à 6, contenant en outre d'autres excipients appropriés choisis parmi les agents de réticulation, les activateurs de pénétration, les conservateurs, les plastifiants, les parfums, les antioxydants et les émollients.

8. Patch selon l'une quelconque des revendications 1 à 7, dans lequel la couche de support est transparente, semi-occlusive ou occlusive, perméable à l'oxygène.

9. Patch selon la revendication 8, dans lequel la couche de support est un film d'éther ou d'ester de polyuréthane, un polyéthylène, un film d'acétate de vinyle et d'éthylène ou de polyoléfine avec un MVTR (taux de transmission de la vapeur humide) de 50 à 3 500 g/m²/jour et une épaisseur de 20 à 150 µm.

10. Patch selon l'une quelconque des revendications 1 à 9, dans lequel le solvant très volatil avec un point d'ébullition non supérieur à 100°C est choisi parmi l'eau, l'éthanol, l'acétone, l'isopropanol, l'acide acétique, l'isobutanol, l'acétate d'éthyle, la méthyl éthyl cétone.
